# EUROPEAN PATENT SPECIFICATION

(11) **EP 3 991 649 B1**
(45) Date of publication and mention of the grant of the patent: **10.12.2025**
(21) Application number: 20204116.6
(22) Date of filing: 27.10.2020
(51) Int. Cl.: A61B 5/11, A61B 5/00

(54) **NON-OBTRUSIVE GAIT MONITORING METHODS AND SYSTEMS FOR REDUCING RISK OF FALLING**
UNAUFFÄLLIGES GANGÜBERWACHUNGSVERFAHREN UND SYSTEME ZUR VERMINDERUNG DES STURZRISIKOS
SYSTÈMES ET PROCÉDÉS DE SURVEILLANCE DE MARCHE NON INTRUSIFS POUR RÉDUIRE LE RISQUE DE CHUTE

(43) Date of publication of application: 04.05.2022
(73) Proprietor: SHFT II ApS, 1264 Copenhagen K (DK)
(72) Inventor: Motzfeldt, Tony, 1264 Copenhagen K (DK); Barfred, Stefan, 1264 Copenhagen K (DK); Kelager, Micky, 1264 Copenhagen K (DK)
(74) Representative: Nordic Patent Service A/S

(56) References cited:
- WO-A1-2009/147597
- WO-A1-2016/110804
- WO-A1-2016/141380
- US-A1- 2009 137 933
- US-A1- 2018 177 436
- US-A1- 2018 264 320

## Description

### TECHNICAL FIELD

The disclosure relates to monitoring and analyzing motion of people using wearable sensors. In particular, the embodiments described herein relate to methods and systems for non-obtrusive gait monitoring for identifying fall events and calculating risk of falling, and for providing dynamic feedback through artificial intelligence-based coaching.

### BACKGROUND

Gait monitoring using wearable sensors is a relatively new field, wherein the number of newly developed technologies have been increasing substantially in the recent years. These technologies include inertial measurement units (IMUs) to provide information for calculating stride frequency, stride length, and similar metrics; pressure sensors; and more complex wearable systems with IMUs and pressure, bend, and height sensors, which often leads to bulky, impractical implementations. A few systems also have wireless communication mounted in-sole of a shoe, while the majority have external pods with Internet-of-Things (IoT), power, and wireless data transmission.

One prior art example is US20180177436A1 which describes a system for remote monitoring for elderly fall prediction, detection, and prevention that relies on collecting and analyzing sensor data including kinematic activity data such as accelerometer data or gyroscopic data at a biomechanical sensing device coupled to a user; generating a fall risk assessment; and detecting a trigger condition and triggering a response to the fall risk assessment.

Another prior art example is WO2016110804A1 which describes a system comprising wearable devices attached or applied to limbs, body, head or other body extremities. The system enables diagnostic or prognostic monitoring of relevant parameters and corresponding analysis applicable to the onset or detection of events or health conditions of interest. One application relates to fall risk prediction using motion sensors.

The direct prevention of an older person to fall depends on a multitude of physiological, behavioral, and environmental factors. Some of the identified risk factors by different studies include gait, gait changes, and posture. For wearable fall intervention systems, the trade-off between the amount and quality of sensors and range of power and/or IoT technology for reliable gait analysis clashes with the need for a non-obtrusive and affordable solution.

A cost-effective technology for large scale, non-obtrusive screening and monitoring of gait changes aimed for predicting and preventing fall events does not yet exist, despite a clear and evidenced need. In particular, there is a need for a gait detection technology that enables monitoring key biomechanical risk factors in real-time and real settings; developing assessment models combining biomechanical inputs with key biometric variables to create profiles for high risk walking performance; providing real-time, non-technical feedback and guidance to users, and supporting fast rehabilitation.

Furthermore, the technology should also provide fast analysis of the gait and postural performance and provide clear feedback to the user.

In addition, for older and/or limited capability adults it is crucial to lower the communication barrier and provide clear, respectful, and correct two-way information, and to overcome the technology adoption barrier, as well to provide it in a manner that is non-obtrusive, convenient, comfortable, and socially acceptable.

### SUMMARY

It is an object to provide a method and system for dynamic, non-obtrusive monitoring of locomotion of a person for identifying fall events and calculating risk of falling, and thereby solving or at least reducing the problems mentioned above.

The foregoing and other objects are achieved by the features of the independent claims. Further implementation forms are apparent from the dependent claims, the description, and the figures.

According to a first aspect, there is provided a computer-implemented method for dynamic, non-obtrusive monitoring of locomotion of a person, the method comprising:
obtaining, by a wearable communication unit, at least one sensor signal comprising a temporal sequence of sensor data from at least one wearable sensor unit arranged to measure locomotion of a person;
processing, by a signal processing unit of the wearable communication unit, the sensor signal to extract biometric data relating to locomotion of the person;
transmitting the biometric data to a mobile device;
analyzing the biometric data, using a machine learning-based risk prediction algorithm executed on a processor of the mobile device or called by the processor of the mobile device from a remote server, to identify patterns related to falling of the person; and
identifying a fall event or calculating risk of falling of the person based on the identified patterns.

*With this method it becomes possible to provide a non-obtrusive monitoring of a person for identifying fall events and calculating risk of falling, using only an already available and in-use mobile device (such as a smartphone or smart watch) and small-sized wearable sensors pre-arranged e.g. on* or *in the shoes of the person.*

*The extracted biometric data and sensor signals can, on their own or in combination, signal that a fall is about to happen, is happening, or has just happened, and such situation is a high priority. Using a machine learning model, additional calculations can enhance fall predictions while also prevent false positives.*

*By applying the trained machine learning-based risk prediction algorithm in the described manner it becomes possible to estimate the fall probability of users and to identify a fall event during their gait cycles in real-time.*

*Thus, the described method provides an affordable solution for fall intervention for both supervised and independent use by identifying gait issues that are directly correlated with adverse or dangerous health conditions, specifically but not limited to falling, and to create awareness and novel practices in fall intervention and connected health.*

*This goal is achieved in particular by providing a system designed for two-way humanized coaching via a highly customizable and adaptable mechanism based on deep learning algorithms that can adjust* to *the situation, conditions, and in particular to the individual user's behavior and preferences.*

In a possible implementation form of the first aspect the method further comprises transmitting a warning based on the identified fall event or the calculated risk of falling to a predefined second person (e.g. in an emergency response center), together with location data of the person obtained from the mobile device.

*Should a true fall occur, this method enables the phone or smart watch to call the local emergency dispatch center and automatically, with a clear message, will tell the operator about the incident and provide the GPS coordinates required to locate the user.*

In a further possible implementation form of the first aspect the at least one wearable sensor unit comprises at least one motion sensor, and the sensor signal comprises temporal sequences of motion data.

In a further possible implementation form of the first aspect the at least one wearable sensor unit comprises two motion sensors, each motion sensor configured to be attached to a foot of a user, and an inter-foot distance measurement system configured to measure inter-foot distance based on the position of the two motion sensors, wherein the sensor signal further comprises temporal sequences of inter-foot distance data.

In an embodiment the inter-foot distance measurement system comprises at least one of an ultrasound-based system, a radio-frequency based system, a magnetic field-based system, or a visual system comprising a light source and stereo cameras.

In a further possible implementation form of the first aspect each motion sensor comprises an inertial measurement unit, (IMU) and the sensor signal comprises at least one of 3-axis linear acceleration, 3-axis angular velocity, and 3-axis orientation data. In some embodiments the IMU comprises at least one of an accelerometer, a gyroscope, and a magnetometer.

In an embodiment the at least one wearable sensor unit further comprises at least one local pressure sensor, and wherein the sensor signal further comprises temporal sequences of local pressure data.

In another possible embodiment the method further comprises obtaining additional sensor data from at least one of a barometer sensor or a location sensor of the mobile device, and wherein the sensor signal further comprises temporal sequences of at least one of a barometer sensor data or location sensor data.

In a further possible implementation form of the first aspect processing the sensor signal comprises at least one of filtering, smoothing, normalizing, and aggregating into time slots of equal size by the signal processing unit before transmitting to the mobile device.

In an embodiment processing the sensor signal (further) comprises fusing multiple temporal sequences of sensor data by the signal processing unit before transmitting to the mobile device.

In another possible embodiment processing the sensor signal (further) comprises time-stamping the temporal sequence of sensor data by the signal processing unit before transmitting to the mobile device.

In an embodiment, the biometric data is transmitted by the wearable communication unit to a mobile device using a long-range, low power consumption wireless protocol. In a possible embodiment the wearable communication unit is configured to compressing and transmitting data using at least one of a Bluetooth, GPS, and narrowband IoT signal at 27 to 380 kb/second at a power consumption of 25 to 100 mW.

In a further possible implementation form of the first aspect the extracted biometric data comprises at least one of:
- inter-foot distance,
- stride length and frequency (stride count per minute),
- single contact time and double contact time,
- center of body displacement, and
- stride and step variability.

In a further possible implementation form of the first aspect identifying patterns related to falling of the person further comprises analyzing a combination of the biometric data and at least one type of sensor data extracted from the sensor signal.

In a further possible implementation form of the first aspect the machine learning-based risk prediction algorithm comprises a pre-trained neural network using data collected from test persons wearing at least one wearable sensor unit while performing gait cycles.

In a possible embodiment the test persons comprise at least one of a group of persons with no history of falling, a group of persons with a history of falling one or more times, and a group of persons falling while the data is collected.

In a possible embodiment of the test persons comprise a group of virtual persons anatomically modelled using physics-based modelling and animation techniques wearing virtual sensor units and performing simulated falls.

In a possible embodiment the neural network is a Recurrent Neural Network (RNN) or a Multilayer Perceptron Network.

In a further possible implementation form of the first aspect the machine learning-based risk prediction algorithm is trained to identify patterns in the biometric data within the context of different scenario parameters, the scenario parameters comprising at least one of
- static scenario parameters based on location data extracted from a location sensor (GPS) of the mobile device (such as house, hospital, nursery home, etc.), or
- adaptable scenario parameters based on dynamically obtained sensory data (such as light condition, indoor or outdoor environment, current weather, gait conditions (flat or stairs), etc.).

In a further possible implementation form of the first aspect the machine learning-based risk prediction algorithm is trained to identify patterns in the biometric data within the context of different user parameters, the user parameters comprising at least one of
- static user parameters based on predefined user data (such as age, condition, given preferences), or
- adaptable user parameters based on detected behavioral changes of the person based on comparing biometric data extracted from sensor signals obtained real-time to existing records of biometric data of the same person, wherein the existing records may be obtained via self-screening or supervised screening.

In a further possible implementation form of the first aspect the method further comprises:
- determining a feedback, using a rule-based or machine learning-based artificial intelligence algorithm executed on a processor of the mobile device or called by the processor of the mobile device from a remote server, based on the identified fall event or the calculated risk of falling; and
- presenting the feedback to the person on a user interface of the mobile device.

*Using such a rule-based or machine learning-based artificial intelligence algorithm (such as supervised learning models combined with reinforcement learning) it becomes possible to coach the users, in a personal and friendly manner, to improve their gait cycles to prevent falling and subsequently to avoid injuries.*

In a further possible implementation form of the first aspect determining the feedback is further based on a personalized training plan called by the rule-based or machine learning-based artificial intelligence algorithm, the personalized training plan comprising a set of actions with assigned execution dates; and presenting the feedback comprises presenting at least one action assigned to a date of determining the feedback.

In a possible embodiment the personalized training plan is auto-generated using a machine learning-based artificial intelligence algorithm, based on user-specific information, such as static user parameters (age, condition, given preferences), or adaptable user parameters (detected behavioral changes of a person).

In a further possible implementation form of the first aspect identifying patterns in the biometric data comprises comparing biometric data extracted from sensor signals obtained in real-time to existing records of biometric data of the same person; and the feedback comprises a personalized message based on a change in performance in accordance with the results of the comparison.

In a possible embodiment, if the identified pattern in the biometric data indicates a decrease in biometric parameters with respect to at least one of gait, balance, or posture, the feedback comprises a personalized message designed to improve performance of the person.

In a possible embodiment, if the identified pattern in the biometric data indicates an increase in biometric parameters with respect to at least one of gait, balance, or posture, the feedback comprises a personalized message designed to encourage the person to maintain or further increase the biometric parameters.

In a further possible implementation form of the first aspect determining the feedback comprises:
- receiving a natural language-based user input comprising a request regarding a biometric parameter of the person;
- analyzing the user input using a natural language processing algorithm to identify a portion of the biometric data of the person related to the request; and
- determining a natural language-based output in response to the user input based on the respective portion of the biometric data using a natural language processing algorithm.

In a further possible implementation form of the first aspect the method further comprises identifying follow-up patterns in the biometric data by comparing follow-up biometric data extracted from sensor signals after presenting a feedback to the person to expected biometric data determined based on the personalized training plan; and determining, using a reinforcement learning based algorithm, a follow-up feedback to be presented to the person.

In a further possible implementation form of the first aspect the method further comprises:
- providing a conversational user interface implemented on a touch screen display of the mobile device;
- receiving the user input through the conversational user interface by detecting a touch input of the person; and
- presenting the determined output in response to the user input through the conversational user interface.

In another possible implementation form of the first aspect the method further comprises:
- providing an audio input-output interface on the mobile device;
- receiving the user input through the audio input-output interface as a spoken input; and
- presenting the determined output in response to the user input through the audio input-output interface in an audio format.

In another possible implementation form of the first aspect the method further comprises:
- detecting a behavioral change pattern of the person based on comparing biometric data extracted from sensor signals obtained real-time to existing records of biometric data of the same person; and
- automatically adjusting at least one of the personalized training plan or the rule-based or machine learning-based artificial intelligence algorithm based on the behavioral change pattern of the person.

In another possible implementation form of the first aspect the method further comprises:
- detecting user input through the user interface of the mobile device in response to the feedback, the user input comprising at least one control parameter; and
- updating at least one of the personalized training plan or the rule-based or machine learning-based artificial intelligence algorithm based on the control parameter.

According to a second aspect, there is provided a system for dynamic, non-obtrusive monitoring of locomotion of a person, the system comprising:
- at least one wearable sensor unit arranged to measure locomotion of a person and to generate a sensor signal comprising a temporal sequence of sensor data;
- a wearable communication unit configured to obtain a sensor signal, to process the sensor signal using a signal processing unit to extract biometric data relating to locomotion of the person, and to transmit the biometric data; and
- a mobile device comprising:
- a processor configured to analyze, using a machine learning-based risk prediction algorithm executed on the processor or called by the processor from a remote server, the biometric data to identify patterns related to falling and to identify a fall event or calculate risk of falling of the person based on the identified patterns according to any one of the possible implementation forms of the first aspect; and
- a user interface configured to present feedback to the person based on the identified fall event or the calculated risk of falling.

According to a third aspect, there is provided a computer program product, encoded on a computer-readable storage device, operable to cause a system according to the second aspect to perform operations according to the methods of any one of the possible implementation forms of the first aspect.

These and other aspects will be apparent from and the embodiment(s) described below.

### BRIEF DESCRIPTION OF THE DRAWINGS

In the following detailed portion of the present disclosure, the aspects, embodiments, and implementations will be explained in more detail with reference to the example embodiments shown in the drawings, in which:
Fig. 1 shows a flow diagram of a method for identifying a fall event or calculating risk of falling of a person in accordance with the first aspect, using a system in accordance with the second aspect;
Fig. 2 shows an overview the main components of a system in accordance with a possible implementation of the second aspect;
Fig. 3 illustrates different types of biometric data determined in accordance with a possible implementation of the first aspect;
Fig. 4 shows a flow chart of training a machine learning-based risk prediction algorithm in accordance with a possible implementation of the first aspect;
Fig. 5 shows a flow chart of determining a warning and/or a feedback in accordance with a possible implementation of the first aspect;
Fig. 6 illustrates different types of input parameters of a neural network in accordance with a possible implementation of the first aspect;
Fig. 7 shows a flow chart of determining a follow-up feedback in accordance with a possible implementation of the first aspect;
Fig. 8 illustrates a conversational user interface implemented in accordance with a possible implementation of the first aspect; and
Fig. 9 shows block diagram of a method for identifying a fall event or calculating risk of falling of a person in accordance with the first aspect, using a system in accordance with the second aspect.

### DETAILED DESCRIPTION

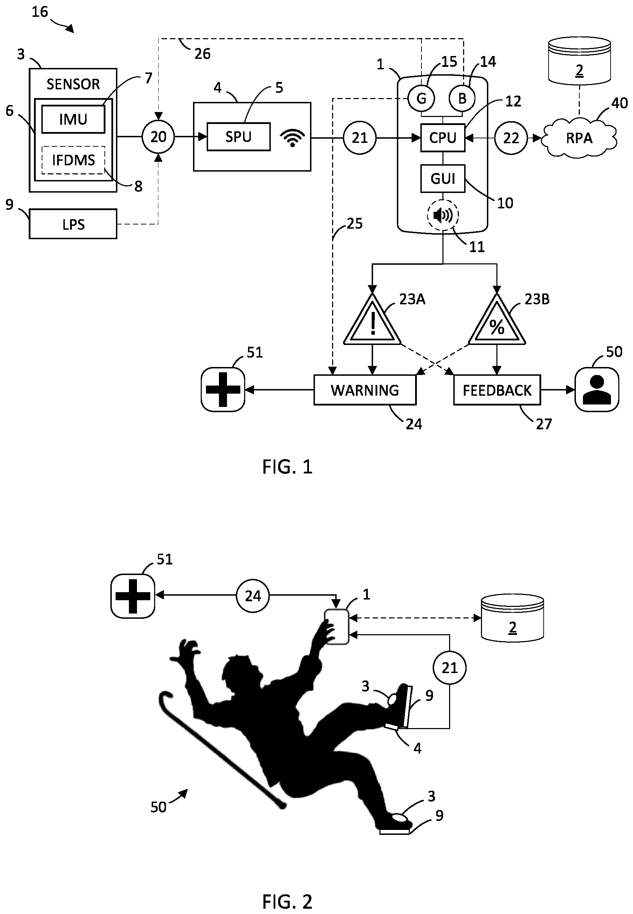

Fig. 1 shows a flow diagram of a method for identifying a fall event 23A or calculating risk of falling 23B of a person 50 in accordance with the present disclosure, using a computer-based system 16 such as for example the system shown on Fig. 2.

The system 16 comprises at least one wearable sensor unit 3 arranged to measure locomotion of a person 50 and to generate a sensor signal 20 comprising a temporal sequence of sensor data.

In an embodiment, the system 16 comprises at least one motion sensor 6, and the sensor signal 20 comprises temporal sequences of motion data.

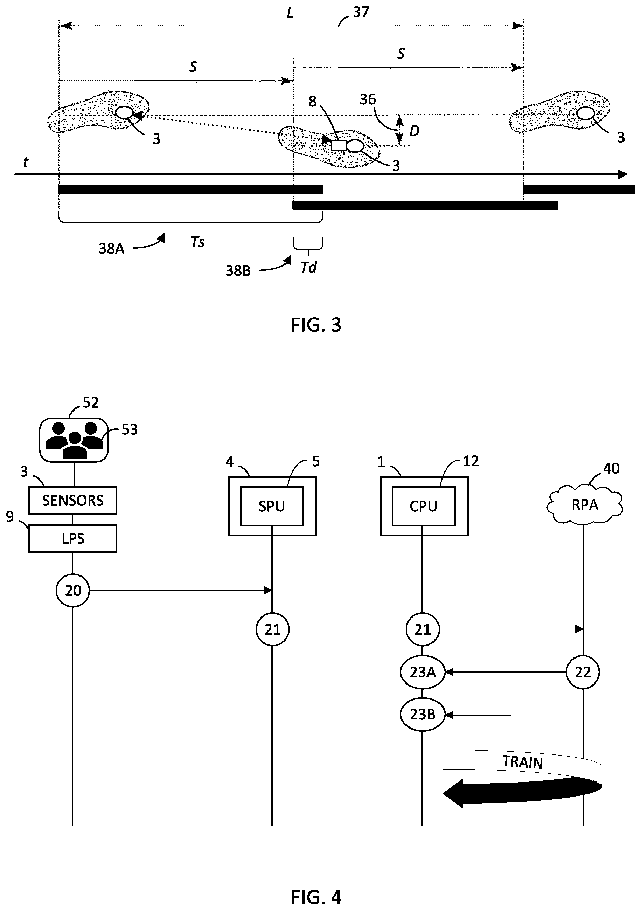

In a possible embodiment, the system 16 comprises two wearable sensor units 3, which can be motion sensors 6, each wearable sensor unit 3 configured to be attached to a foot of a user, and an inter-foot distance measurement system 8 configured to measure at least an inter-foot distance 36 biometric based on the position of the two motion sensors 6, as shown in Fig. 3. In this embodiment the sensor signal 20 comprises temporal sequences of inter-foot distance data.

The inter-foot distance measurement system 8 may comprises an ultrasound-based system, a radio-frequency based system (such as tracking distance by measuring radio signal strength), a magnetic field-based system, or a visual system comprising a light source and stereo cameras.

Each motion sensor 6 may comprise an inertial measurement unit 7, in which case the sensor signal 20 comprises at least one of 3-axis linear acceleration, 3-axis angular velocity, and 3-axis orientation data.

In possible embodiments, the system 16 may further comprise at least one local pressure sensor 9, in which case the sensor signal 20 further comprises temporal sequences of local pressure data. The local pressure sensors 9 may comprise several graphene pressure sensors (e.g. 12) embedded in a flexible sensor pad configured to be arranged in the sole of a shoe.

In possible embodiments, the system 16 may further comprise at least one of a barometer sensor 14 or a location sensor 15 arranged in the mobile device 1. In such embodiments, as also illustrated in Fig. 1, the method further comprises obtaining additional sensor data 26 from at least one of a barometer sensor 14 or a location sensor 15, and the sensor signal 20 further comprises temporal sequences of at least one of a barometer sensor data or location sensor data.

The system 16 further comprises a wearable communication unit 4 configured to obtain a sensor signal 20, to process the sensor signal 20 using a signal processing unit 5 to extract biometric data 21 relating to locomotion of the person 50, and to transmit the biometric data 21, e.g. using a long-range, low power consumption wireless protocol.

In a possible embodiment extracted biometric data 21 comprises at least one of:
- inter-foot distance 36 measured by an inter-foot distance measurement system 8,
- stride length 37 and frequency measured e.g. by motion sensors 6 attached to the feet of the person 50,
- single contact time 38A and double contact time 38B measured e.g. by motion sensors 6 or local pressure sensors 9 attached to or arranged at the feet of the person 50,
- center of body displacement measured e.g. by motion sensors 6 attached to the body of the person 50, and
- stride and step variability.

These possible biometric data 21 measurements are illustrated in Fig. 3 using a schematic top view of steps taken by a person 50, wherein contact times of each foot are projected to a timeline for determining single and double contact times.

In possible embodiments, processing the sensor signal 20 may comprise filtering, smoothing, normalizing, and/or aggregating into time slots of equal size by the signal processing unit 5 before transmission.

In some embodiments, processing the sensor signal 20 further comprises fusing multiple temporal sequences of sensor data by the signal processing unit 5 before transmitting to the mobile device 1.

In some embodiments, processing the sensor signal 20 further comprises time-stamping the temporal sequence of sensor data by the signal processing unit 5 before transmitting to the mobile device 1.

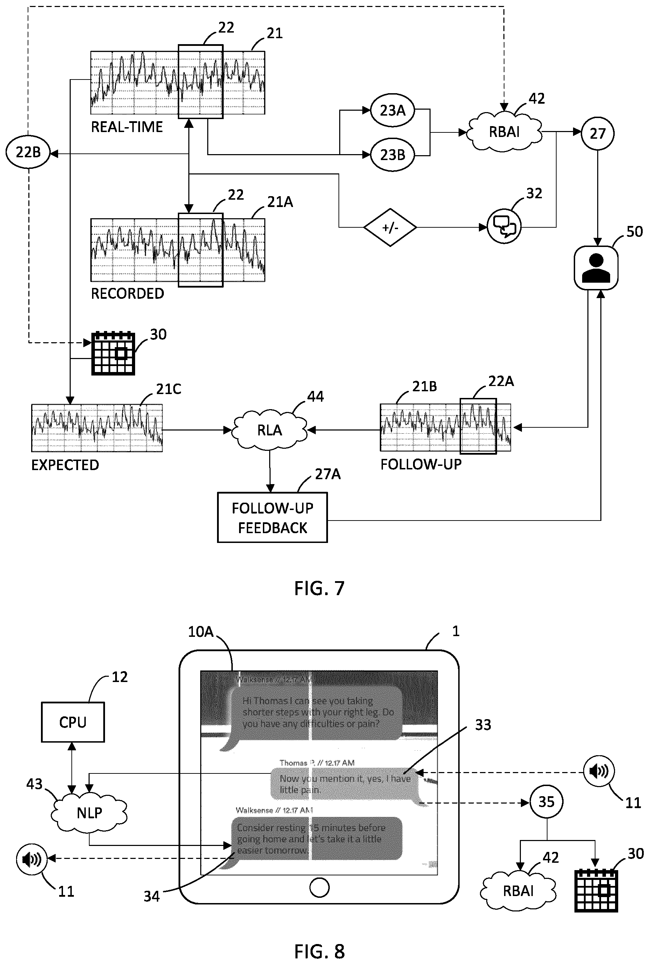

The biometric data 21 may be transmitted to a mobile device 1, such as a smartphone or smart watch, comprising a processor 12 configured to analyze, using a machine learning-based risk prediction algorithm 40, the biometric data 21 to identify patterns 22 related to falling and to identify a fall event 23A or calculate risk of falling 23B of the person 50 based on the identified patterns 22, as illustrated in Fig. 7.

In some embodiments, the step of identifying patterns 22 related to falling of the person 50 further comprises analyzing a combination of the biometric data 21 and at least one type of raw sensor data extracted from the sensor signal 20.

In some embodiments, the wearable communication unit 4 is configured to compressing and transmitting data using at least one of a Bluetooth, GPS, and narrowband IoT signal at 27 to 380 kb/second at a power consumption of 25 to 100 mW.

The machine learning-based risk prediction algorithm 40 may be executed on the processor 12 or called by the processor 12 from a remote server 2.

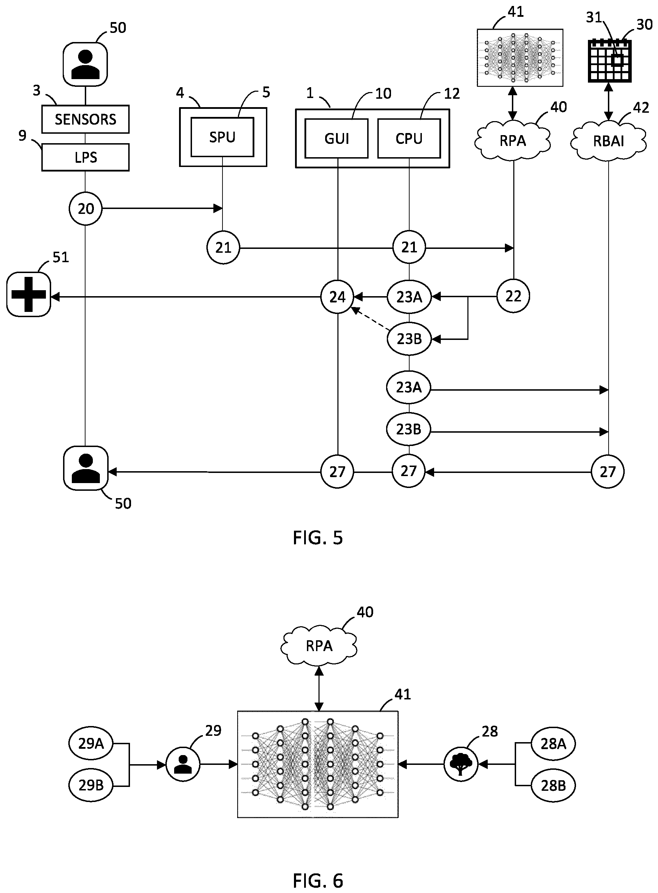

In some embodiments, the machine learning-based risk prediction algorithm 40 comprises at least one model (such as a neural network 41 illustrated in Fig. 6) pre-trained using data collected from test persons 52 wearing at least one wearable sensor unit 3 while performing gait cycles, as illustrated in Fig. 4, wherein results from risk prediction algorithm 40 based on test persons 52 are compared to expected results and fed back to train the model(s).

In an embodiment, the test persons 52 may comprise at least one of a group of persons with no history of falling, a group of persons with a history of falling one or more times, and a group of persons falling while the data is collected.

The model may be trained on data collected from test persons 52 with wearable sensor units 3 placed on each foot and one wearable sensor unit 3 placed on the chest. The test persons 52 may be asked to walk on a treadmill in a controlled lab to record their gait cycles.

In an embodiment, the test persons may (further) comprise a group of virtual persons 53 anatomically modelled using physics-based modelling and animation techniques wearing virtual sensor units and performing simulated falls. Using physics-based modelling and animation techniques it becomes possible to affect the virtual persons 53 in several ways, including slippery surfaces, pushes, heavy wind, instability in balance, etc. Verified simulated falls can then be used as data sources and data can be collected from the same body positions as from the real test persons 52.

As illustrated in Fig. 1 and 5, the system may also comprise a user interface 10 configured to present a feedback 27 to the person 50 based on the identified fall event 23A or the calculated risk of falling 23B.

In an embodiment, a warning 24 may also be generated based on the identified fall event 23A or the calculated risk of falling 23B, and transmitted automatically to a predefined second person 51 (e.g. in an emergency response center) either in case of an actual fall event 23A or if a calculated risk of falling 23B exceeds a predetermined threshold, together with location data 25 of the person 50 obtained from the mobile device 1.

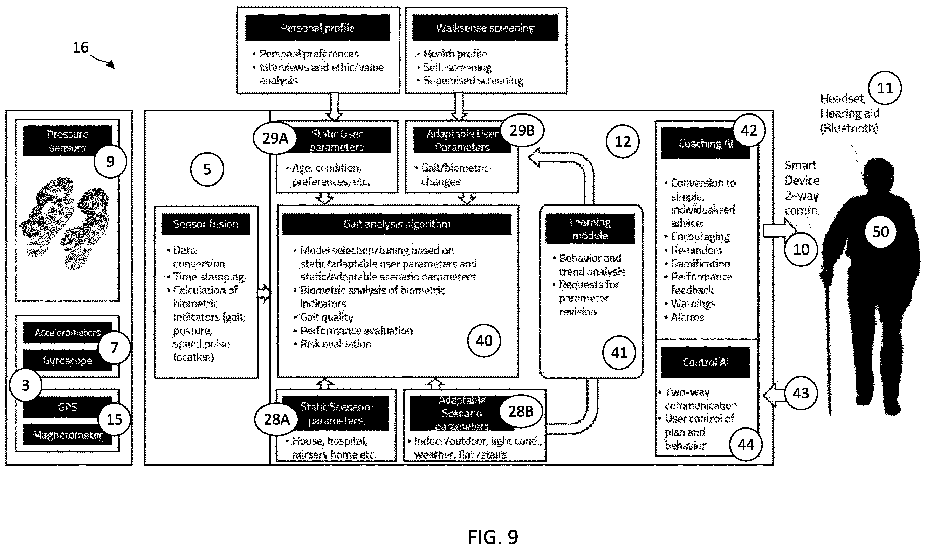

As illustrated in Fig. 6 and 9, the model (s) used by the machine learning-based risk prediction algorithm 40 (such as a neural network 41) may be trained to identify patterns 22 in the biometric data 21 within the context of different scenario parameters 28 and/or different user parameters 29. In possible embodiments the used neural network 41 is a Recurrent Neural Network (RNN). In other possible embodiments the used neural network 41 is a Multilayer Perceptron Network.

In some embodiments, the scenario parameters 28 may comprise at least one of the following:
- static scenario parameters 28A based on location data 25 extracted from a location sensor 15 (GPS) of the mobile device 1 (such as house, hospital, nursery home, etc.), or
- adaptable scenario parameters 28B based on dynamically obtained sensory data, such as light condition, indoor or outdoor environment, current weather, or gait conditions (e.g. flat or stairs).

In some embodiments, the user parameters 29 may comprise at least one of the following:
- static user parameters 29A based on predefined user data, such as age, condition, given preferences, or
- adaptable user parameters 29B based on detected behavioral changes of the person 50 based on comparing biometric data 21 extracted from sensor signals 20 obtained real-time to existing records of biometric data 21A of the same person 50, wherein the existing records may be obtained via self-screening or supervised screening.

As illustrated in Fig. 7, the method may comprise determining a feedback 27, using a rule-based or machine learning-based artificial intelligence algorithm 42, based on the identified fall event 23A or the calculated risk of falling 23B; and presenting the feedback 27 to the person 50 on a user interface 10 of the mobile device 1. The rule-based or machine learning-based artificial intelligence algorithm 42 may be executed on a processor 12 of the mobile device 1 or called by the processor 12 of the mobile device 1 from a remote server 2.

In some embodiments, as also illustrated in Fig. 7, determining the feedback 27 is further based on a personalized training plan 30 called by the rule-based or machine learning-based artificial intelligence algorithm 42. The personalized training plan 30 can be any type of regimen generated for a person 50, and may comprise a set of actions 31 with assigned execution dates. In such embodiments, presenting the feedback 27 comprises presenting at least one action 31 assigned to a date of determining the feedback 27.

As also illustrated in Fig. 7, identifying patterns 22 in the biometric data 21 may comprise comparing biometric data 21 extracted from sensor signals 20 obtained in real-time to existing records of biometric data 21A of the same person 50. In such embodiments, the feedback 27 may comprise a personalized message 32 based on a change in performance in accordance with the results of the comparison.

For example, if the identified pattern in the biometric data 21 indicates a decrease in biometric parameters with respect to at least one of gait, balance, or posture, the feedback 27 may comprise a personalized message 32 designed to improve performance of the person 50. If the identified pattern in the biometric data 21 however indicates an increase in biometric parameters with respect to at least one of gait, balance, or posture, the feedback 27 may comprise a personalized message 32 designed to encourage the person 50 to maintain or further increase the biometric parameters.

As further illustrated in Fig. 7, the method may further comprise identifying follow-up patterns 22A in the biometric data 21 by comparing follow-up biometric data 21B extracted from sensor signals 20 after presenting a feedback 27 to the person 50 to expected biometric data 21C determined based on the personalized training plan 30. In this case, a follow-up feedback 27A may be determined, using a reinforcement learning based algorithm 44, to be presented to the person 50 in return.

As further illustrated in Fig. 7, the method may further comprise detecting a behavioral change pattern 22B of the person 50 based on comparing biometric data 21 extracted from sensor signals 20 obtained real-time to existing records of biometric data 21A of the same person 50; and automatically adjusting at least one of the personalized training plan 30 or the rule-based or machine learning-based artificial intelligence algorithm 42 based on the behavioral change pattern 22B of the person 50.

Fig. 8 illustrates a possible implementation of a conversational user interface 10A on a touch screen display of the mobile device 1. In this exemplary embodiment, user input 33 may be received through the conversational user interface 10A in a text format by detecting a touch input of the person 50 (e.g. in response to a preset conversation-starter message sent to the person 50), and a determined output 34 may be then presented in response to the user input 33 through the conversational user interface 10A in a text format.

In an embodiment, the user input 33 may be a natural language-based user input 33, e.g. comprising a request regarding a biometric parameter of the person 50. In such an embodiment, the user input 33 is analyzed using a natural language processing algorithm 43 to identify a portion of the biometric data 21 of the person 50 related to the request; and a natural language-based output 34 is generated in response, based on the respective portion of the biometric data 21 using a natural language processing algorithm 43.

In a possible embodiment, an audio input-output interface 11 may further be provided on the mobile device 1, such as a wired or wireless headset, or hearing aid. In such cases user input 33 may be received through the audio input-output interface 11 as a spoken input; and the determined output 34 can be transmitted in response to the user input 33 through the audio input-output interface 11 in an audio format.

In some embodiments, as also illustrated in Fig. 8, a user input 33 can be detected through the user interface 10 of the mobile device 1 in response to a feedback 27, in which case the user input 33 may comprise at least one of control parameter 35. In such embodiments, the method may comprise updating the personalized training plan 30 and/or the rule-based or machine learning-based artificial intelligence algorithm 42 based on the control parameter 35 provided.

Fig. 9 illustrates an exemplary embodiment of a system 16 in accordance with the present disclosure, wherein steps and features that are the same or similar to corresponding steps and features previously described or shown herein are denoted by the same reference numeral as previously used for simplicity.

The various aspects and implementations have been described in conjunction with various embodiments herein. However, the invention is defined by the appended claims.

## Claims

1. A computer-implemented method for dynamic, non-obtrusive monitoring of locomotion of a person (50), the method comprising:
obtaining, by a wearable communication unit (4), at least one sensor signal (20) comprising a temporal sequence of sensor data from at least one wearable sensor unit (3) arranged to measure locomotion of a person (50);
the at least one wearable sensor unit (3) comprising at least one motion sensor (6), and at least one local pressure sensor (9) attached to or arranged at the feet of the person (50), and wherein the sensor signal (20) comprises temporal sequences of motion data and temporal sequences of local pressure data;processing, by a signal processing unit (5) of the wearable communication unit (4), the sensor signal (20) to extract biometric data (21) relating to locomotion of the person (50), wherein the extracted biometric data (21) comprises single contact time (38A) and double contact time (38B) measured by the at least one local pressure sensor (9);
transmitting the biometric data (21) to a mobile device (1);
analyzing the biometric data (21), using a machine learning-based risk prediction algorithm (40) executed on a processor (12) of the mobile device (1) or called by the processor (12) of the mobile device (1) from a remote server (2), to identify patterns (22) related to falling of the person (50); and
identifying a fall event (23A) or calculating risk of falling (23B) of the person (50) based on the identified patterns (22).

2. The method according to claim 1, wherein the at least one local pressure sensor (9) comprises graphene pressure sensors embedded in a flexible sensor pad configured to be arranged in the sole of a shoe.

3. The method according to any one of claims 1 or 2, wherein the method further comprises transmitting a warning (24) based on the identified fall event (23A) or the calculated risk of falling (23B) to a predefined second person (51), together with location data (25) of the person (50) obtained from the mobile device (1).

4. A method according to any one of claims 1 to 3, wherein the at least one wearable sensor unit (3) comprises two motion sensors (6), each motion sensor (6) configured to be attached to a foot of a user, and an inter-foot distance measurement system (8) configured to measure inter-foot distance (36) based on the position of the two motion sensors (6), wherein the sensor signal (20) further comprises temporal sequences of inter-foot distance data.

5. A method according to any one of claims 1 to 4, wherein each motion sensor (6) comprises an inertial measurement unit (7), and the sensor signal (20) comprises at least one of 3-axis linear acceleration, 3-axis angular velocity, and 3-axis orientation data.

6. A method according to any one of claims 1 to 5, wherein processing the sensor signal (20) comprises at least one of filtering, smoothing, normalizing, and aggregating into time slots of equal size by the signal processing unit (5) before transmitting to the mobile device (1).

7. A method according to any one of claims 1 to 6, wherein said extracted biometric data (21) comprises at least one of:
- inter-foot distance (36),
- stride length (37) and frequency,
- center of body displacement, and
- stride and step variability.

8. A method according to any one of claims 1 to 7, wherein identifying patterns (22) related to falling of the person (50) further comprises analyzing a combination of said biometric data (21) and at least one type of sensor data extracted from said sensor signal (20).

9. The method according to any one of claims 1 to 8, wherein said machine learning-based risk prediction algorithm (40) comprises a neural network (41) pre-trained using data collected from test persons (52) wearing at least one wearable sensor unit (3) while performing gait cycles.

10. The method according to any one of claims 1 to 9, wherein the method further comprises
determining a feedback (27), using a rule-based or machine learning-based artificial intelligence algorithm (42) executed on a processor (12) of the mobile device (1) or called by the processor (12) of the mobile device (1) from a remote server (2), based on the identified fall event (23A) or the calculated risk of falling (23B); and
presenting the feedback (27) to the person (50) on a user interface (10) of the mobile device (1).

11. A method according to claim 10, wherein determining the feedback (27) is further based on a personalized training plan (30) called by the rule-based or machine learning-based artificial intelligence algorithm (42), the personalized training plan (30) comprising a set of actions (31) with assigned execution dates; and
wherein presenting the feedback (27) comprises presenting at least one action (31) assigned to a date of determining the feedback (27).

12. A method according to any one of claims 10 or 11, wherein the method further comprises identifying follow-up patterns (22A) in the biometric data (21) by comparing follow-up biometric data (21B) extracted from sensor signals (20) after presenting a feedback (27) to the person (50) to expected biometric data (21C) determined based on the personalized training plan (30); and
determining, using a reinforcement learning based algorithm (44), a follow-up feedback (27A) to be presented to the person (50).

13. A method according to any one of claims 10 to 12, wherein the method further comprises
detecting a behavioral change pattern (22B) of the person (50) based on comparing biometric data (21) extracted from sensor signals (20) obtained real-time to existing records of biometric data (21A) of the same person (50); and
automatically adjusting at least one of the personalized training plan (30) or the rule-based or machine learning-based artificial intelligence algorithm (42) based on the behavioral change pattern (22B) of the person (50).

14. A system (16) for dynamic, non-obtrusive monitoring of locomotion of a person, the system (16) comprising:
at least one wearable sensor unit (3) arranged to measure locomotion of a person (50) and to generate a sensor signal (20), the at least one wearable sensor unit (3) comprising at least one motion sensor (6), and at least one local pressure sensor (9) attached to or arranged at the feet of the person (50), and the sensor signal (20) comprising temporal sequences of motion data and temporal sequences of local pressure data;
a wearable communication unit (4) configured to obtain a sensor signal (20), to process the sensor signal (20) using a signal processing unit (5) to extract biometric data (21) relating to locomotion of the person (50), and to transmit the biometric data (21), the biometric data (21) comprising single contact time (38A) and double contact time (38B) measured by the at least one local pressure sensor (9); and
a mobile device (1) comprising
a processor (12) configured to analyze, using a machine learning-based risk prediction algorithm (40) executed on the processor (12) or called by the processor (12) from a remote server (2), the biometric data (21) to identify patterns (22) related to falling and to identify a fall event (23A) or calculate risk of falling (23B) of the person (50) based on the identified patterns (22) according to any one of the claims 1 to 13; and
a user interface (10) configured to present feedback (27) to the person (50) based on the identified fall event (23A) or the calculated risk of falling (23B).

15. A computer program product encoded on a computer-readable storage device (13), operable to cause a system (16) according to claim 14 to perform operations according to the methods of any one of claims 1 to 13.

## Patentansprüche

1. Computerumgesetztes Verfahren zur dynamischen, unauffälligen Überwachung der Fortbewegung einer Person (50), wobei das Verfahren umfasst:
Erzielen, durch eine anziehbare Kommunikationseinheit (4), mindestens eines Sensorsignals (20), das eine Zeitsequenz von Sensordaten umfasst, von mindestens einer anziehbaren Sensoreinheit (3), die dazu eingerichtet ist, die Fortbewegung einer Person (50) zu messen;
wobei die mindestens eine anziehbare Sensoreinheit (3) mindestens einen Bewegungssensor (6) und mindestens einen lokalen Drucksensor (9), der an den Füssen der Person (50) angebracht oder eingerichtet ist, umfasst, und wobei das Sensorsignal (20) Zeitsequenzen von Bewegungsdaten und Zeitsequenzen von lokalen Druckdaten umfasst;
Verarbeiten, durch eine Signalverarbeitungseinheit (5) der anziehbaren Kommunikationseinheit (4), des Sensorsignals (20), um biometrische Daten (21) über die Fortbewegung einer Person (50) zu extrahieren, wobei die extrahierten biometrischen Daten (21) eine Einzelkontaktzeit (38A) und eine Doppelkontaktzeit (38B) umfassen, die von dem mindestens einen lokalen Drucksensor (9) gemessen werden;
Senden der biometrischen Daten (21) an eine mobile Vorrichtung (1);
Analysieren der biometrischen Daten (21), unter Verwendung eines Risikovorhersagealgorithmus (40), der auf maschinellem Lernen basiert und auf einem Prozessor (12) der mobilen Vorrichtung (1) ausgeführt wird oder durch einen Prozessor (12) der mobilen Vorrichtung (1) von einem entfernten Server (2) abgerufen wird, um Muster (22) zu identifizieren, die mit einem Sturz der Person (50) zusammenhängen; und
Identifizieren eines Sturzereignisses (23A) oder Berechnen des Sturzrisikos (23B) der Person (50) auf der Grundlage der identifizierten Muster (22).

2. Verfahren nach Anspruch 1, wobei der mindestens eine lokale Drucksensor (9) Graphendrucksensoren umfasst, die in einem biegsamen Sensorplättchen integriert sind, dass dazu konfiguriert ist, in der Sohle eines Schuhs eingerichtet zu werden.

3. Verfahren nach einem der Ansprüche 1 oder 2, wobei das Verfahren ferner das Senden einer Warnung (24) basierend auf dem identifizierten Sturzereignis (23A) oder dem berechneten Sturzrisiko (23B) an eine vordefinierte zweite Person (51) zusammen mit Standortdaten (25) der Person (50), die von der mobilen Vorrichtung (1) erzielt werden, umfasst.

4. Verfahren nach einem der Ansprüche 1 bis 3, wobei die mindestens eine anziehbare Sensoreinheit (3) zwei Bewegungssensoren (6), wobei jeder Bewegungssensor (6) dazu konfiguriert ist, an einem Fuß eines Benutzers angebracht zu werden, und ein System (8) zum Messen eines Zwischenfußabstands, das dazu konfiguriert ist, den Abstand zwischen den Füssen (36) auf der Grundlage der Position der beiden Bewegungssensoren (6) zu messen, umfasst, wobei das Sensorsignal (20) ferner Zeitsequenzen von Daten über einen Zwischenfußabstand umfasst.

5. Verfahren nach einem der Ansprüche 1 bis 4, wobei jeder Bewegungssensor (6) eine Trägheitsmesseinheit (7) umfasst, und das Sensorsignal (20) mindestens eines von einer 3-achsigen linearen Beschleunigung, einer 3-achsigen Winkelgeschwindigkeit und 3-achsigen Orientierungsdaten umfassen.

6. Verfahren nach einem der Ansprüche 1 bis 5, wobei das Verarbeiten des Sensorsignals (20) mindestens eines von Filtern, Glätten, Normieren und Aggregieren in gleich große Zeitschlitze durch die Signalverarbeitungseinheit (5) vor dem Senden an die mobile Vorrichtung (1) umfasst.

7. Verfahren nach einem der Ansprüche 1 bis 6, wobei die extrahierten biometrischen Daten (21) mindestens eines umfassen von:
- dem Zwischenfußabstand (36),
- der Schrittlänge (37) und Frequenz,
- der Verlagerung des Körpermittelpunktes, und
- der Variabilität von Schritt und Tritt.

8. Verfahren nach einem der Ansprüche 1 bis 7, wobei das Identifizieren von Mustern (22), die mit dem Stürzen der Person (50) zusammenhängen, ferner das Analysieren einer Kombination der biometrischen Daten (21) und mindestens einer Art von extrahierten Sensordaten des Sensorsignals (20) umfasst.

9. Verfahren nach einem der Ansprüche 1 bis 8, wobei der Risikovorhersagealgorithmus (40), der auf maschinellem Lernen basiert, ein neuronales Netzwerk (41) umfasst, das unter Verwendung von Daten vortrainiert wird, die bei Testpersonen (52) erhoben werden, die mindestens eine anziehbare Sensoreinheit (3) tragen, während sie Gangzyklen ausführen.

10. Verfahren nach einem der Ansprüche 1 bis 9, wobei das Verfahren ferner umfasst:
Bestimmen einer Rückmeldung (27) unter Verwendung eines Algorithmus mit künstlicher Intelligenz (42), der auf Regeln oder auf maschinellem Lernen basiert und auf einem Prozessor (12) der mobilen Vorrichtung (1) ausgeführt wird oder von einem Prozessor (12) der mobilen Vorrichtung (1) von einem entfernten Server (2) basierend auf dem identifizierten Sturzereignis (23A) oder dem berechneten Sturzrisiko (23B) abgerufen wird; und
Vorlegen der Rückmeldung (27) für die Person (50) an einer Benutzerschnittstelle (10) der mobilen Vorrichtung (1).

11. Verfahren nach Anspruch 10, wobei das Bestimmen der Rückmeldung (27) ferner auf einem personalisierten Trainingsplan (30) basiert, der von dem Algorithmus mit künstlicher Intelligenz (42), der auf Regeln oder maschinellem Lernen basiert, abgerufen wird, wobei der personalisierte Trainingsplan (30) eine Menge von Aktionen (31) mit zugeteilten Ausführungsdaten umfasst; und
wobei das Vorlegen der Rückmeldung (27) das Vorlegen mindestens einer Aktion (31) umfasst, die einem Datum der Bestimmung der Rückmeldung (27) zugewiesen ist.

12. Verfahren nach einem der Ansprüche 10 oder 11, wobei das Verfahren ferner das Identifizieren von Verfolgungsmustern (22A) in den biometrischen Daten (21) durch Vergleichen der biometrischen Verfolgungsdaten (21B), die aus den Sensorsignalen (20) nach dem Vorlegen einer Rückmeldung (27) für die Person (50) extrahiert werden, mit erwarteten biometrischen Daten (21C), die auf der Grundlage eines personalisierten Trainingsplans (30) bestimmt werden; und
Bestimmen unter Verwendung eines Algorithmus (44), der auf Verstärkungslernen basiert, einer Verfolgungsrückmeldung (27A), die der Person (50) vorzulegen ist.

13. Verfahren nach einem der Ansprüche 10 bis 12, wobei das Verfahren ferner umfasst:
Detektieren eines Verhaltensänderungsmusters (22B) der Person (50) auf der Grundlage eines Vergleichs der biometrischen Daten (21), die aus Sensorsignalen (20) extrahiert werden, die in Echtzeit erzielt werden, mit existierenden Aufzeichnungen von biometrischen Daten (21A) der gleichen Person (50); und
automatisches Anpassen mindestens eines von dem personalisierten Trainingsplan (30) oder dem Algorithmus mit künstlicher Intelligenz (42), der auf Regeln oder auf dem maschinellen Lernen basiert, auf der Grundlage des Verhaltensänderungsmusters (22B) der Person (50).

14. System (16) zur dynamischen, unauffälligen Überwachung der Fortbewegung einer Person (50), wobei das System (16) umfasst:
mindestens eine anziehbare Sensoreinheit (3), die eingerichtet ist, um die Fortbewegung einer Person (50) zu messen und ein Sensorsignal (20) zu generieren, wobei die mindestens eine anziehbare Sensoreinheit (3) mindestens einen Bewegungssensor (6) und mindestens einen lokalen Drucksensor (9), der an den Füssen der Person (50) angebracht oder eingerichtet ist, umfasst, und wobei das Sensorsignal (20) Zeitsequenzen von Bewegungsdaten und Zeitsequenzen von lokalen Druckdaten umfasst;
eine anziehbare Kommunikationseinheit (4), die dazu konfiguriert ist, ein Sensorsignal (20) zu erzielen, um das Sensorsignal (20) unter Verwendung einer Signalverarbeitungseinheit (5) zu verarbeiten, um biometrische Daten (21), welche die Fortbewegung der Person (50) betreffen, zu extrahieren, und um die biometrischen Daten (21) zu senden, wobei die biometrischen Daten (21) eine Einzelkontaktzeit (38A) und eine Doppelkontaktzeit (38B) umfassen, die von dem mindestens einen lokalen Drucksensor (9) gemessen werden; und
eine mobile Vorrichtung (1), umfassend
einen Prozessor (12), der dazu konfiguriert ist, unter Verwendung eines Risikovorhersagealgorithmus (40), der auf maschinellem Lernen basiert und auf dem Prozessor (12) ausgeführt wird oder von dem Prozessor (12) von einem Server (2) abgerufen wird, die biometrischen Daten (21) zu analysieren, um Muster (22), die einen Sturz betreffen, zu identifizieren und ein Sturzereignis (23A) zu identifizieren oder ein Sturzrisiko (23B) der Person (50) basierend auf den identifizierten Mustern (22) nach einem der Ansprüche 1 bis 13 zu berechnen; und
eine Benutzerschnittstelle (10), die dazu konfiguriert ist, eine Rückmeldung (27) der Person (50) basierend auf dem identifizierten Sturzereignis (23A) oder dem berechneten Sturzrisiko (23B) vorzulegen.

15. Computerprogrammprodukt, das auf einer computerlesbaren Speichervorrichtung (13) codiert ist, die dazu betriebsfähig ist, zu veranlassen, dass ein System (16) nach Anspruch 14 Arbeitsgänge nach einem der Ansprüche 1 bis 13 ausführt.

## Revendications

1. Procédé mis en œuvre par ordinateur pour la surveillance dynamique et discrète de la motricité d'une personne (50), le procédé comprenant les étapes suivantes consistant à :
obtenir, par une unité de communication portable (4), au moins un signal de capteur (20) comprenant une séquence temporelle de données de capteur d'au moins une unité de capteur portable (3) agencée pour mesurer la motricité d'une personne (50) ;
ladite au moins une unité de capteur portable (3) comprenant au moins un capteur de mouvement (6), et au moins un capteur de pression locale (9) rattaché à ou agencé au niveau des pieds de la personne (50), et dans lequel le signal de capteur (20) comprend des séquences temporelles de données de mouvement et des séquences temporelles de données de pression locale ;
traiter, par une unité de traitement de signal (5) d'une unité de communication portable (4), le signal de capteur (20) pour extraire des données biométriques (21) liées à la motricité de la personne (50), dans lequel les données biométriques extraites (21) comprennent un temps de contact unique (38A) et un temps de contact double (38B) mesurés par ledit au moins un capteur de pression locale (9) ;
envoyer les données biométriques (21) à un dispositif mobile (1) ;
analyser les données biométriques (21), en utilisant un algorithme de prédiction de risque (40) basé sur l'apprentissage machine, exécuté sur un processeur (12) du dispositif mobile (1|) ou appelé par le processeur (12) du dispositif mobile (1) à partir d'un serveur distant (2), pour identifier des motifs (22) liés une chute de la personne (50) ; et
identifier un événement de chute (23A) ou calculer le risque de chute (23B) de la personne (50) sur la base des motifs identifiés (22).

2. Procédé selon la revendication 1, dans lequel ledit au moins un capteur de pression locale (9) comprend des capteurs de pression de graphène incorporés dans une plaquette de capteur flexible configurée pour être agencée dans la semelle d'une chaussure.

3. Procédé selon l'une quelconque des revendications 1 ou 2, dans lequel le procédé comprend en outre l'envoi d'une alerte (24) basée sur l'événement de chute identifié (23A) ou le risque de chute calculé (23B) à une deuxième personne prédéfinie (51) avec des données d'emplacement (25) de la personne (50) obtenues du dispositif mobile (1).

4. Procédé selon l'une quelconque des revendications 1 à 3, dans lequel ladite au moins une unité de capteur portable (3) comprend deux capteurs de mouvement (6), chaque capteur de mouvement (6) étant configuré pour être rattaché à un pied d'un utilisateur, et un système (8) de mesure de distance entre les pieds, configuré pour mesurer la distance entre les pieds (36) sur la base de la position des deux capteurs de mouvement (6), dans lequel le signal de capteur (20) comprend en outre des séquences temporelles de données de distance entre les pieds.

5. Procédé selon l'une quelconque des revendications 1 à 4, dans lequel chaque capteur de mouvement (6) comprend une unité de mesure inertielle (7), et le signal de capteur (20) comprend au moins un élément parmi une accélération linéaire sur 3 axes, une vélocité sur 3 axes et des données d'orientation sur 3 axes.

6. Procédé selon l'une quelconque des revendications 1 à 5, dans lequel le traitement du signal de capteur (20) comprend au moins un élément parmi le filtrage, le lissage, la normalisation et l'agrégation en tranches de temps de taille égale par une unité de traitement de signal (5) avant l'envoi au dispositif mobile (1).

7. Procédé selon l'une quelconque des revendications 1 à 6, dans lequel lesdites données biométriques extraites (21) comprennent au moins un élément parmi :
- la distance entre les pieds (36),
- la longueur de foulée (37) et la fréquence,
- le déplacement du centre du corps, et
- la variabilité de la foulée et du pas.

8. Procédé selon l'une quelconque des revendications 1 à 7, dans lequel l'identification de motifs (22) liés à la chute de la personne (50) comprend en outre l'analyse d'une combinaison desdites données biométriques (21) et au moins un type de données de capteur extraites dudit signal de capteur (20).

9. Procédé selon l'une quelconque des revendications 1 à 8, dans lequel ledit algorithme de prédiction de risque (40) basé sur l'apprentissage machine comprend un réseau neuronal (41) pré-entraîné en utilisant des données prélevées chez des personnes testées (52) portant au moins une unité de capteur portable (3) pendant qu'ils exécutent des cycles de marche.

10. Procédé selon l'une quelconque des revendications 1 à 9, dans lequel le procédé comprend en outre les étapes consistant à
déterminer un retour (27), en utilisant un algorithme d'intelligence artificielle (42) basé sur des règles ou sur un apprentissage machine, exécuté sur un processeur (12) du dispositif mobile (1) ou appelé par un processeur (12) du dispositif mobile (1) à partir d'un serveur distant (2), sur la base de l'événement de chute identifié (23A) ou du risque de chute calculé (23B) ; et
présenter le retour (27) à la personne (50) au niveau d'une interface utilisateur (10) du dispositif mobile (1).

11. Procédé selon la revendication 10, dans lequel la détermination du retour (27) est en outre basée sur un plan d'entraînement personnalisé (30) appelé par l'algorithme d'intelligence artificielle (42) basé sur des règles ou l'apprentissage machine, le plan d'entraînement personnalisé (30) comprenant un ensemble d'actions (31) avec des dates d'exécution attribuées ; et
dans lequel la présentation du retour (27) comprend la présentation d'au moins une action (31) attribuée à une date de détermination du retour (27).

12. Procédé selon l'une quelconque des revendications 10 ou 11, dans lequel le procédé comprend en outre l'identification de motifs de suivi (22A) dans les données biométriques (21) en comparant des données biométriques de suivi (21B) extraites de signaux de capteur (20) après la présentation d'un retour (27) à la personne (50) avec des données biométriques attendues (21C) déterminées sur la base d'un plan d'entraînement personnalisé (30) ; et
déterminer, en utilisant un algorithme basé sur l'apprentissage par renforcement (44), un retour de suivi (27A) à présenter à la personne (50).

13. Procédé selon l'une quelconque des revendications 10 à 12, dans lequel le procédé comprend en outre les étapes consistant à :
détecter un motif de changement de comportement (22B) de la personne (50) sur la base d'une comparaison des données biométriques (21), extraites de signaux de capteur (20) obtenus en temps réel, avec des enregistrements existants de données biométriques (21A) de la même personne (50) ; et
ajuster automatiquement au moins l'un du plan d'entraînement personnalisé (30) ou de l'algorithme d'intelligence artificielle (42) basé sur des règles ou sur l'apprentissage machine, sur la base du motif de changement de comportement (22B) de la personne (50).

14. Système (16) pour la surveillance dynamique et discrète de la motricité d'une personne (50), le système (16) comprenant :
au moins une unité de capteur portable (3), agencée pour mesurer la motricité d'une personne (50) et pour générer un signal de capteur (20), ladite au moins une unité de capteur portable (3) comprenant au moins un capteur de mouvement (6), et au moins un capteur de pression locale (9) rattaché à ou agencé au niveau des pieds de la personne (50), et le signal de capteur (20) comprenant des séquences temporelles de données de mouvement et des séquences temporelles de données de pression locale ;
une unité de communication portable (4) configurée pour obtenir un signal de capteur (20), pour traiter le signal de capteur (20) en utilisant une unité de traitement de signal (5), pour extraire des données biométriques (21) liées à la motricité de la personne (50), et pour envoyer les données biométriques (21), les données biométriques (21) comprenant un temps de contact unique (38A) et un temps de contact double (38B) mesurés par ledit au moins un capteur de pression locale (9); et
un dispositif mobile (1) comprenant
un processeur (12) configuré pour analyser, en utilisant un algorithme de prédiction de risque (40) basé sur un apprentissage machine, exécuté sur un processeur (12) ou appelé par le processeur (12) à partir d'un serveur distant (2), les données biométriques (21) pour identifier des motifs (22) liés à une chute et pour identifier un événement de chute (23A) ou pour calculer un risque de chute (23B) de la personne (50) sur la base des motifs identifiés (22) selon l'une quelconque des revendications 1 à 13 ; et
une interface utilisateur (10) configurée pour présenter un retour (27) à la personne (50) sur la base de l'événement de chute identifié (23A) ou du risque de chute calculé (23B).

15. Produit de programme informatique codé sur un dispositif de stockage (13) lisible par ordinateur, opérationnel pour amener un système (16) selon la revendication 14 à effectuer des opérations selon les procédés selon l'une quelconque des revendications 1 à 13.
